# EUROPEAN PATENT APPLICATION

(11) **EP 3 135 192 A1**
(43) Date of publication of application: **01.03.2017**
(21) Application number: 15783720.4
(22) Date of filing: 23.04.2015
(51) Int. Cl.: A61B 5/05, A61B 5/08, A61B 5/04

(54) **DEVICE FOR IMAGING AND DIAGNOSING UPPER AIRWAY OBSTRUCTION CONDITION USING CONDUCTIVITY TOMOGRAPHY**

(30) Priority: 24.04.2014 KR 20140049095
(71) Applicant: Industry-Academic Cooperation Foundation Gyeongsang National University, Jinju-si, Gyeongsangnam-do 660-701 (KR)
(72) Inventor: KIM, Sang Wook, Jinju-si Gyeongsangnam-do 660-770 (KR); OH, Tong In, Suwon-si Gyeonggi-do 443-811 (KR); KWON, Oh In, Seoul 158-755 (KR); WOO, Eung Je, Seongnam-si Gyeonggi-do 463-845 (KR)
(74) Representative: Botti, Mario
(86) International application number: PCT/KR2015/004061
(87) International publication number: WO 2015/163710

(57) **Abstract**

The present invention relates to a device for imaging and diagnosing the condition of obstruction of the upper airway, which can image the condition of a change in the upper airway of an OSAS patient occurring during natural sleep by using EIT and can extract information required for diagnosis. The device for imaging and diagnosing the condition of obstruction of the upper airway by using EIT according to the present invention does not require high costs, enables repeated examination to be conducted without the risk of exposure to radioactive rays, and enables examination to be performed over a prolonged period of time in an actual natural sleep state, thereby accurately diagnosing the condition of obstruction of the upper airway.

## Description

### Technical Field

The present invention relates to a device for diagnosing obstruction of the upper airway, and more particularly to a device for imaging and diagnosing the condition of obstruction of the upper airway, which can image the condition of a change in the upper airway of an obstructive sleep apnea syndrome patient occurring during natural sleep by using electrical impedance tomography and can extract information required for diagnosis.

### Background Art

Obstructive sleep apnea syndrome (OSAS) is a disease in which complete or partial obstruction of the upper airway occurs repeatedly during sleep.

Since air inhaled for respiration must pass through a soft tissue tube structure called "the upper airway," the condition of obstruction of the upper airway is greatly influenced by the shape of a tube and the fluid mechanical characteristics of air passing through the tube, and thus information about the presence and condition of obstruction of the upper airway may become diagnostic information for the discovery of a fundamental cause and the performance of treatment related to OSAS corresponding to most sleep apnea. Sleep apnea is defined as a case where apnea, which is a symptom in which respiration stops for 10 or more seconds, or hypopnea, in which a respiratory air flow decreases by 30% or more for 10 or more seconds and an oxygen saturation reduction of 3% or more or arousal is entailed, occurs five or more times per hour. According to the results of foreign and domestic research, although OSAS is frequently developed in adult males, the prevalence rate of OSAS and the severity of the symptoms of OSAS increase in proportion to age regardless of gender. OSAS is also significantly related to the degree of obesity that is represented by a body mass index (BMI).

OSAS causes various complications, such as cardiovascular disease, endocrine disease, neuropsychiatric disease, sexual dysfunction, etc. Therefore, OSAS brings about an increase in health and medical expenses resulting from the occurrence of medical complications, and also brings about an increase in social and economic expenses resulting from the occurrence of traffic and industrial accidents attributable to daytime hypersomnolence.

In order to overcome these problems, various methods for treating OSAS have been proposed. That is, current methods for treating OSAS are basically classified into three types of methods, i.e., continuous positive airway pressure (CPAP), surgery, and oral appliances, in addition to the weight control of an obese patient.

CPAP is a device that removes obstruction of the upper airway by artificially injecting positive-pressure air through the nose. CPAP exhibits a success rate equal to or higher than 90% and thus has a success rate higher than the other methods, but has a problem in that there are many cases where patients stop treatment due to the inconvenience in which the patients must sleep in the state of wearing equipment. Furthermore, the surgical treatment method suffers from difficulty in selecting an appropriate surgery target patient and in analyzing the cause of the failure of surgery due to the absence of an imaging examination method capable of diagnosing the condition of obstruction of the upper airway of a patient in a natural sleep state. Among the oral applications, an appliance that has been clinically used so far is a mandibular advancement device (MAD). The MAD is designed to widen a space behind the base of tongue region. Although a treatment success rate can be increased when a patient whose upper airway obstruction chiefly occurs in a region behind the base of tongue region is selected and the MAD is applied to the patient, the MAD also suffers from difficulty in finding the cause of the success or failure of treatment due to the absence of an examination method capable of diagnosing a change in the upper airway during natural sleep before and after the treatment.

The treatment method using CPAP has the highest success rate, but has a high treatment discontinuance rate due to serious inconvenience. The surgery and the MAD exhibit low treatment success rates due to the absence of a method for diagnosing the condition of obstruction of the upper airway during natural sleep. Therefore, there arises a need for a method for diagnosing whether the upper airway has been obstructed and the condition of a change in obstruction in a natural sleep state.

Of course, conventionally, there have been various imaging methods for diagnosing the condition of obstruction of the upper airway. For example, there have been examination methods, such as sleep videofluoroscopy (SVF), cine-MRI, drug-induced sleep endoscopy (DISE), etc. However, these imaging examination methods are all examination methods that are designed to induce artificial sleep through the administration of a sedative and to be performed within a short time of about 30 minutes. These imaging examination methods are limited in that induced sleep is different from natural sleep. Furthermore, SVF provides a low image precision due to its two-dimensional planar examination, cannot evaluate the lateral collapse of the upper airway, and is limited in terms of repeated examination due to unavoidable exposure to radioactive rays. Although cine-MRI provides a high image precision, it cannot evaluate the overall upper airway region at the same time because it cannot three-dimensionally and concurrently acquire upper airway images at different levels, and it is chiefly used for research purposes and cannot be generally used for the evaluation of an OSAS patient because of its high expenses. The use of DISE is gradually increasing because exposure to radioactive rays can be avoided. However, DISE disturbs the natural sleep of a patient during examination due to the stimuli of an endoscope to the upper airway. In particular, when multi-level obstruction is caused by various anatomical structures, the visual field of measurement is limited, and thus DISE cannot evaluate the overall condition of obstruction.

As a result, the region of obstruction of the upper airway of an OSAS patient cannot be accurately determined in a natural sleep state over a prolonged period of time by using the conventional examination methods, and thus the condition of obstruction of the upper airway of the OSAS patient cannot be accurately diagnosed. The condition of obstruction of the upper airway of an OSAS patient varies depending on an individual, and thus there arises a need for a technology for diagnosing the condition of obstruction of the upper airway, which can be practiced in a natural sleep state to diagnose a difference for each individual.

### Disclosure

### Technical Problem

The present invention is intended to overcome the above-described problems, and an object of the present invention is to provide a diagnostic device, which does not require high costs and enables examination to be conducted over a prolonged period of time without the risk of exposure to radioactive rays, thereby effectively examining the condition of obstruction of the upper airway occurring during natural sleep over the course of a few hours.

### Technical Solution

According to an embodiment of the present invention, there is provided a device for imaging and diagnosing the condition of obstruction of the upper airway by using electrical impedance tomography, the device including: a conductivity signal measurement unit configured to measure the distribution of electric potentials that changes in accordance with a change in the shape of the upper airway; a feature extraction unit configured to restore conductivity images of an upper airway by using data on the measured change in the distribution of electric potentials and extract feature information from the restored conductivity images; and an image and diagnostic information output unit configured to display the extracted feature information or restored conductivity images.

Preferably, the device further includes a multichannel electrode interface including multichannel electrodes attached onto face surfaces around the region of the upper airway; and the conductivity signal measurement unit measures the change in the distribution of electric potentials by applying current via the multichannel electrode interface and measuring voltage induced by the applied current.

Preferably, the feature extraction unit extracts a change in the area of the region of the upper airway or the geometrical feature of the region of the upper airway by using a feature extraction algorithm.

Preferably, the feature extraction unit performs the removal of noise by using the data on the change in the distribution of electric potentials measured through monitoring.

Preferably, the feature extraction unit performs the removal of noise by using the pattern analysis of sectional data or the average value of the feature information.

Preferably, the conductivity signal measurement unit measures the distribution of electric potentials at high speed so that the conductivity images of the upper airway are restored at a rate of 50 to 100 images per second.

Preferably, the image and diagnostic information output unit synchronizes the feature information or the conductivity images, corresponding to a plurality of layers, with each other, and then provides them.

Preferably, the image and diagnostic information output unit provides diagnostic information corresponding to the feature information based on a learning-based database that stores data on treatment instances of obstructive sleep apnea syndrome.

According to another embodiment of the present invention, there is provided a device for imaging and diagnosing the condition of obstruction of the upper airway by using electrical impedance tomography, the device including: a multichannel electrode interface; and an imaging device configured to restore conductivity images by using current-voltage data transferred from the multichannel electrode interface; wherein the multichannel electrode interface includes multichannel electrodes attached onto face surfaces around the region of the upper airway, and a protective gear configured in a shape that surrounds outsides of the multichannel electrodes.

Preferably, the multichannel electrodes include conductive fiber electrodes manufactured based on Ag-plated elastic fiber or PVDF nanofiber web.

Preferably, the multichannel electrodes are arranged in a three-dimensional array including a plurality of layers.

### Advantageous Effects

The device for imaging and diagnosing the condition of obstruction of the upper airway by using EIT according to the present invention does not require high costs, allows repeated examination to be conducted without the risk of exposure to radioactive rays, and enables examination to be performed over a prolonged period of time in an actual natural sleep state, thereby accurately diagnosing the condition of obstruction of the upper airway.

The device for imaging and diagnosing the condition of obstruction of the upper airway by using EIT according to the present invention performs fast measurement capable of restoring 50 to 100 conductivity images per second, thereby more accurately detecting an upper airway obstruction occurring over a short period of time.

The device for imaging and diagnosing the condition of obstruction of the upper airway by using EIT according to the present invention performs the removal of noise by using upper airway obstruction data repeatedly generated during monitoring in a natural sleep state, thereby improving the accuracy of measurement.

The device for imaging and diagnosing the condition of obstruction of the upper airway by using EIT according to the present invention provides information about the condition of obstruction of the upper airway measured three-dimensionally, thereby achieving more accurate and effective diagnosis.

The device for imaging and diagnosing the condition of obstruction of the upper airway by using EIT according to the present invention provides variable data extracted from conductivity images measured over a prolonged period of time corresponding to actual sleep time, thereby providing information or data useful for the treatment of OSAS without a need to see and analyze conductivity images generated over a prolonged period of time.

### Description of Drawings

Figs. 1a and 1b are views showing examples of a multichannel electrode interface that is attached onto the upper airway region;
Fig. 2 is a diagram showing the configuration of a device for imaging and diagnosing the condition of obstruction of the upper airway by using electrical impedance tomography according to the present invention;
Figs. 3a and 3b are diagrams illustrating examples of an operation in which a feature extraction unit extracts the geometrical feature of an image of an upper airway region by using a feature extraction algorithm;
Fig. 4 is a graph showing changes in a feature information value over time, which are extracted by the feature extraction unit by using the feature extraction algorithm;
Fig. 5 shows conductivity images restored from data measured at the times at which the upper airway was opened and obstructed, respectively; and
Fig. 6 is a graph showing the time-varying data of conductivity values restored from voltage data measured in a situation in which opening and obstruction of the upper airway were repeated.

### Best Mode

According to an embodiment of the present invention, there is provided a device for imaging and diagnosing the condition of obstruction of the upper airway by using electrical impedance tomography, the device including: a conductivity signal measurement unit configured to measure the distribution of electric potentials that changes in accordance with a change in the shape of the upper airway; a feature extraction unit configured to restore conductivity images of an upper airway by using data on the measured change in the distribution of electric potentials and extract feature information from the restored conductivity images; and an image and diagnostic information output unit configured to display the extracted feature information or restored conductivity images.

Preferably, the device further includes a multichannel electrode interface including multichannel electrodes attached onto face surfaces around the region of the upper airway; and the conductivity signal measurement unit measures the change in the distribution of electric potentials by applying current via the multichannel electrode interface and measuring voltage induced by the applied current.

Preferably, the feature extraction unit extracts a change in the area of the region of the upper airway or the geometrical feature of the region of the upper airway by using a feature extraction algorithm.

Preferably, the feature extraction unit performs the removal of noise by using the data on the change in the distribution of electric potentials measured through monitoring.

Preferably, the feature extraction unit performs the removal of noise by using the pattern analysis of sectional data or the average value of the feature information.

Preferably, the conductivity signal measurement unit measures the distribution of electric potentials at high speed so that the conductivity images of the upper airway are restored at a rate of 50 to 100 images per second.

Preferably, the image and diagnostic information output unit synchronizes the feature information or the conductivity images, corresponding to a plurality of layers, with each other, and then provides them.

Preferably, the image and diagnostic information output unit provides diagnostic information corresponding to the feature information based on a learning-based database that stores data on treatment instances of obstructive sleep apnea syndrome.

According to another embodiment of the present invention, there is provided a device for imaging and diagnosing the condition of obstruction of the upper airway by using electrical impedance tomography, the device including: a multichannel electrode interface; and an imaging device configured to restore conductivity images by using current-voltage data transferred from the multichannel electrode interface; wherein the multichannel electrode interface includes multichannel electrodes attached onto face surfaces around the region of the upper airway, and a protective gear configured in a shape that surrounds outsides of the multichannel electrodes.

Preferably, the multichannel electrodes include conductive fiber electrodes manufactured based on Ag-plated elastic fiber or PVDF nanofiber web.

Preferably, the multichannel electrodes are arranged in a three-dimensional array including a plurality of layers.

### Mode for Invention

The present invention employs Electrical Impedance Tomography (EIT). EIT refers to a technology that is designed to apply low current to a human body via electrodes attached onto surfaces of a human body and image the distribution of internal conductivities by measuring voltage data induced by the applied current. Since the conductivity and dielectric constant of air are significantly different from those of surrounding biological tissues such as a bone, a muscle, fat, a skin, etc., the upper airway has a considerably desirable measurement condition under which changes in the structure and shape of the upper airway can be extracted based on changes in conductivity. Generally, although the image quality of image data acquired via EIT is lower than those of data acquired via other imaging examination methods, EIT may be considered as an appropriate method because it is more important for the diagnosis of OSAS to diagnose whether the upper airway has been obstructed and the condition of the obstruction during long-time natural sleep in a non-invasive manner.

A preferred embodiment of a device for imaging and diagnosing the condition of obstruction of the upper airway by using EIT according to the present invention will be described in detail below with reference to the accompanying drawings.

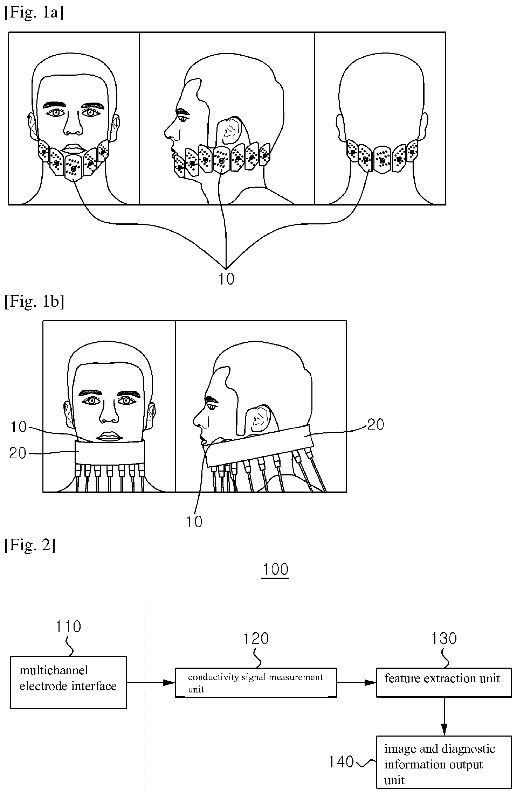

Figs. 1a and 1b are views showing examples of a multichannel electrode interface that is attached onto the upper airway region.

Referring to Fig. 1a, multichannel electrodes 10 are attached onto face surfaces around the upper airway region. Referring to Fig. 1b, to protect the attached electrodes 10, a belt- or mask-shaped protective gear 20 configured in a shape that surrounds the outsides of the electrodes 10 may be worn. The protective gear 20 having the above shape functions to reduce the level of pressure which a user (patient) may feel during the natural sleep and, simultaneously, to maintain the level of contact at which induced voltage can be measured. The level of pressure a user may suffer is significantly reduced compared to that in the case of conventional ultrasonic probes. Accordingly, whether the upper airway of a user (patient) has been obstructed can be easily measured over a prolonged period of time in a natural sleep state by using the multichannel electrode interface having the above structure. In an embodiment, the multichannel electrodes 10 include conductive fiber electrodes manufactured based on Ag-plated elastic fiber or PVDF nanofiber web. A conductive fiber-based dry electrode interface has advantages in that skin reaction to long-time measurement is low and the electrode interface can be formed in various shapes.

Meanwhile, the multichannel electrode interface may be configured as mask- or belt-shaped array electrodes. For example, the distribution of electric potentials on surfaces near the upper airway may be effectively measured through a change of an electrode array and measurement structure in a manner in which 16-, 32-, 64-, or 256-channel electrodes are arranged two-dimensionally or three-dimensionally. In an embodiment, the multichannel electrode interface is configured such that electrodes are arranged in a three-dimensional array including a plurality of layers and conductivity images corresponding to the respective layers can be provided, thereby providing more accurate and effective diagnosis than a conventional method that provides only a two-dimensional sectional image at a specific location. Furthermore, the multichannel electrode interface may provide a switching module that is capable of independent multichannel measurement using high-density array electrodes having various structures. By using the high-density array electrodes, the spatial resolution of conductivity images can be improved, and a change in the distribution of surface electric potentials attributable to a change in internal conductivity can be measured with high sensitivity.

Here, the multichannel electrode interface of the present invention is not necessarily limited to the mask- or belt-shaped array electrodes. In addition, an array multichannel electrode interface having a different shape or structure may be desirably applied by taking into account the level of contact at which the pressure which is sensed by a wearer during natural sleep can be minimized and, simultaneously, the efficiency of data measurement can be increased.

Each electrode of the multichannel electrode interface is used to apply high-frequency current having a relatively low current value, e.g., a value equal to or lower than 1 mA, which cannot be sensed by a patient and to measure induced voltage. The current-voltage data measured via the electrodes is used to extract the shape of the upper airway via an imaging algorithm.

Fig. 2 is a diagram showing the configuration of a device for imaging and diagnosing the condition of obstruction of the upper airway by using EIT according to the present invention.

Referring to Fig. 2, a conductivity signal measurement unit 120 measures a change in the distribution of electric potentials attributable to a change in the shape of the upper airway by using a multichannel electrode interface 110. As described above, the multichannel electrode interface 110 may include a mask- or belt-shaped, arrayed 16-, 32-, 64- or 256-channel electrodes, and may include a belt- or mask-shaped protective gear for protecting the electrodes. The conductivity signal measurement unit 120 measures a change in the distribution of surface electric potentials in regions around the upper airway by applying current within a safe range to a human body at a plurality of frequencies and measuring voltage, induced by the applied current, at a plurality of frequencies via the multichannel electrode interface 110. In an embodiment, the current may employ frequencies in a range between 10 Hz to 1 MHz.

The conductivity signal measurement unit 120 may employ a low-noise signal measurement method configured to measure a small change in the voltage of a living body in order to support a high-sensitive electric potential imaging technology. To maintain the precision of measurement, the regular application of precise and stable current and the stable amplification of voltage induced by the applied current are required, and the voltage gain of a multichannel measurement module must be kept uniform at all measurement frequencies. Accordingly, in an embodiment, the conductivity signal measurement unit 120 may improve the precision of measurement by performing applied current circuit calibration adapted to make the characteristics of all electronic circuits for generating applied current uniform, voltage gain and phase calibration adapted to make characteristics of a voltage measurement circuit uniform, and DC offset calibration.

Furthermore, to perform high-resolution electric potential imaging, a multichannel measurement module using high-density array electrodes may be provided. In an embodiment, the conductivity signal measurement unit 120 may rapidly measure data so that 50 to 100 images can be restored per second in order to accurately detect an upper airway obstruction occurring over a short period of time. For this purpose, the conductivity signal measurement unit 120 uses the multichannel measurement module, and individual modules may perform in sequence a series of signal processing steps, such as the application of current, the measurement of voltage, and the demodulation and transmission of measured voltage, etc., to be sequentially performed by using a pipelining technology.

The feature extraction unit 130 restores images based on the data measured by the conductivity signal measurement unit 120, performs signal processing adapted to improve the quality of the measured data using data measured over a prolonged period of time, and extracts feature information about the condition of obstruction of the upper airway based on the restored images.

The feature extraction unit 130 restores conductivity images using the change in the distribution of electric potentials measured via the conductivity signal measurement unit 120. As described above, the change in the distribution of electric potentials is measured as current-voltage data that is measured by the conductivity signal measurement unit 120 via the multichannel electrode interface 110. To restore the conductivity images, a sensitivity matrix used in common EIT technology is used. An image restoration matrix is calculated by obtaining the inverse matrix of the sensitivity matrix. Here, to improve the quality of the image restoration matrix, mathematical techniques, such as regularization or truncated singular value decomposition (TSVD), may be employed. The conductivity images are calculated through multiplication of an image restoration matrix prepared in advance and the measured voltage data. In an embodiment, the number of pixels of each of the conductivity images may be 4096, 16385, or 65536, and, accordingly, restoration to a 64x64, 128×128, or 256×256 image may be achieved.

The feature extraction unit 130 may employ time-difference and frequency-difference conductivity imaging methods in order to restore images from electric potential distribution measurement data. The time-difference conductivity imaging method is adapted to perform image restoration by using the difference between voltage data measured upon opening or obstruction of the upper airway and voltage data measured at a different time. The frequency-difference conductivity imaging method is adapted to perform image restoration by using the difference between voltage data at one frequency and voltage data at another frequency, among voltage data at a plurality of frequencies measured at the same time. The feature extraction unit 130 may simultaneously employ both the time-difference conductivity imaging method and the frequency-difference conductivity imaging method in order to restore a more elaborate image. In an embodiment, the feature extraction unit 130 may restore conductivity images of the upper airway region from the data measured by the conductivity signal measurement unit 120 at a fast rate of 50 to 100 images per second in order to more accurately detect an upper airway obstruction occurring over a short period of time.

The feature extraction unit 130 may improve the quality of data by removing noise by using a large amount of electric potential distribution data measured via monitoring over the course of several hours. For example, the feature extraction unit 130 may remove noise based on the analysis of an interference signal pattern attributable to the presence of obstruction and movement of the upper airway, or the analysis of the level and frequency of obstruction over time. Since the data measured during several hours of natural sleep is influenced by a change in conductivity attributable to a blood flow, an activity, such as the swallowing of saliva, and respiration, the movement of a patient, and an external interference signal, or the like, the process of removing noise is required.

One method for removing noise is a method using the analysis of the pattern of interval data. First, the feature extraction unit 130 detects the time at which the upper airway is obstructed by detecting that measured voltage data is reduced due to an air area's reduction. Data collected over a predetermined period of time may be separated into a plurality of pieces of data corresponding to respective intervals based on the times at which obstruction of the upper airway has occurred repeatedly. In each of the separate pieces of interval data, all voltage data must decrease during a period of time for which the upper airway changes from an opened state to an obstructed state, and all voltage data must increase during a period of time for which the upper airway changes from an obstructed state to an opened state. The feature extraction unit 130 may effectively remove noise by determining if each interval data has the measured values of a pattern that violates the above-described principle due to the influence of various types of noise and an interference signal, and, if so, excluding such interval data from subsequent processing as low-quality data.

Another method for removing noise is a method using the average value of feature information. The feature extraction unit 130 selects desirable-quality interval data from among a plurality of pieces of voltage data separated based on the times at which the upper airway has been obstructed, and extracts feature information from the selected interval data. In an embodiment, the above-described pattern analysis method may be employed to select desirable-quality interval data. In an embodiment, the feature extraction unit 130 may employ a feature extraction algorithm to be described later in order to extract feature information, and the feature information may include information about a change in the shape and the area of the upper airway during obstruction. Based on the feature information of the desirable-quality interval data extracted as described above, noise may be removed by excluding the interval data, collected over a predetermined period of time, from subsequent data processing when the difference between the value of feature information extracted from such interval data and the average value of the feature information of desirable-quality interval data previously measured is out of a predetermined range.

The feature extraction unit 130 may extract feature information, including the presence and shape of obstruction of the upper airway, a change in the area of the upper airway, and the frequency and level of the obstruction, from the restored conductivity images. For example, the level of a change in the area ranging from 0 to 100%, the frequency of obstruction regarding the average number of obstructions per second, and a change in a closed shape during the occurrence of the obstruction may be extracted. More specifically, by using the conductivity images, information about the size of the upper airway region may be extracted, and also a change in the size of the upper airway region may be tracked over time. Furthermore, feature information indicative of a change in the shape of the upper airway region may be extracted as information in a more useful form, such as a numerical or graph form, by using an algorithm for extracting the geometrical features of a cross section.

The feature extraction unit 130 may employ various feature extraction algorithms in order to extract features, such as the shape of obstruction, the area of the upper airway, etc. For example, the standard deviation of each pixel value may be calculated from a plurality of images generated during the upper airway obstruction occurs repeatedly, and pixels having standard deviations equal to or higher than a predetermined reference value may be defined as the upper airway region. The area of the upper airway region may be calculated from the number of pixels inside the upper airway region defined as described above. Accordingly, a change in the upper airway area may be estimated via a change in the number of pixels inside the upper airway region, from which the presence, frequency and level of obstruction of the upper airway may be extracted.

Furthermore, the feature extraction unit 130 may extract the geometrical feature of an image of the upper airway region by using the feature extraction algorithm. For example, variables for estimating the shape of the upper airway region and a change in the shape may be extracted by setting axes in the lateral and antero-posterior directions of the image of the defined upper airway region and then measuring the lengths of the image of the upper airway region based on the set axis. In this case, the lengths of the image of the upper airway region based on the axes may be measured by a method of calculating the number of pixel groups having the longest length among pixels arranged in parallel with each of the corresponding axes. In some cases, variables capable of representing a region having a more complicated shape may be extracted by using a plurality of lateral or antero-posterior direction axes.

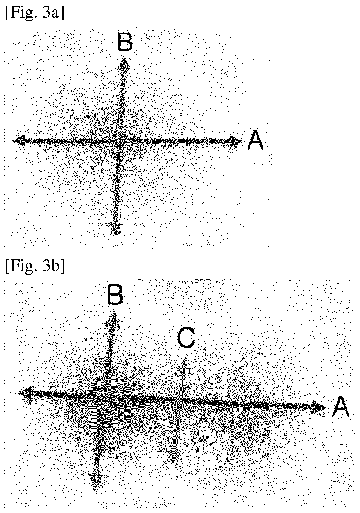

Figs. 3a and 3b are diagrams illustrating examples of an operation in which the feature extraction unit extracts the geometrical feature of an image of an upper airway region by using the feature extraction algorithm.

Referring to Fig. 3a, the feature extraction unit may extract feature information based on the geometrical feature of the upper airway region by measuring the lengths of the upper airway region corresponding to the lateral axis A and antero-posterior axis B of a conductivity image. The feature information based on the geometrical feature of the upper airway region may provide important diagnostic information to a user. For example, when a change in the length in the antero-posterior axis B has occurred prior to and more greatly than a change in the length in the lateral axis A, a user can become aware that the stenosis of a region related to movement in the direction of the antero-posterior axis B may cause the occurrence of obstruction of the upper airway. Referring to Fig. 3b, the geometrical feature of the upper airway region having a more complicated shape may be extracted by using not only a lateral axis A but also a plurality of antero-posterior axes B and C. In an embodiment, the directions of the plurality of axes may be configured to lie at an angle other than a right angle.

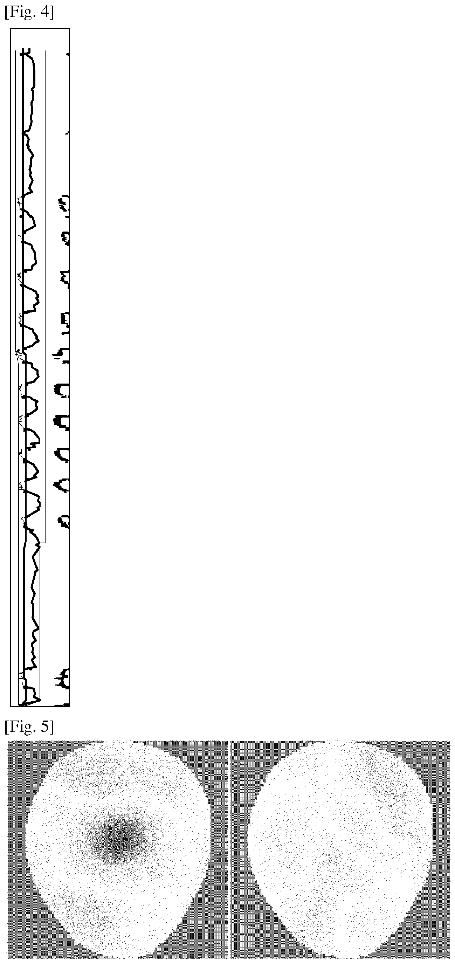

Fig. 4 is a graph showing changes in a feature information value over time, which are extracted by the feature extraction unit by using the feature extraction algorithm.

Referring to Fig. 4, the graph may show feature information (for example, A, B, C, and area information), adapted to determine the presence of obstruction and the feature of the shape of the upper airway, over time. A user may interpret the correlations between a change in the anatomical structure of the upper airway region and an image by analyzing, when obstruction occurs, whether A and B have been reduced at the same rate, or whether A had been reduced at a higher rate first and then B has been reduced after a certain period of time, or the like via the graph. That is, a user can easily obtain useful diagnostic information from the feature information, such as the frequency of obstruction of the upper airway, a change of the area of the upper airway, and the pattern of the shapes of contraction of the upper airway, which cannot be obtained through the simple viewing of an image with an unaided eye. In particular, in view of the facts that the resolutions of EIT images are relatively low and all changes having occurred in a long-time natural sleep state cannot be effectively identified via only images, the present invention can effectively provide information required for diagnosis to a user via feature information.

In an embodiment, the feature extraction unit 130 stores data measured during several hours via monitoring, performs a noise removal process by processing all collected data at once when a predetermined period of time has elapsed, and then extracts the presence, frequency, level and shape of obstruction of the upper airway, thereby improving the accuracy of data.

Referring back to Fig. 2, the image and diagnostic information output unit 140 functions to display feature information, including variables, such as the presence, frequency and shape of obstruction of the upper airway and a change in the area of the upper airway, extracted by the feature extraction unit 130, to a user. In an embodiment, when a three-dimensional array multichannel electrode interface is used, the image and diagnostic information output unit 140 may synchronize conductivity images corresponding to a plurality of layers or a plurality of pieces of feature information extracted from the conductive images with each other, and then may provide them. A user may determine the location (for example, a space behind the soft palate, or a space behind the base of the tongue) of a region generating the cause of obstruction and the direction and level of obstruction by three-dimensionally analyzing a location (an obstruction level, a antero-posterior collapse, a lateral collapse, or circumferential collapse) and also analyzing a process in which obstruction starts and is developed over time by using the above information. In an embodiment, the image and diagnostic information output unit 140 may include a learning-based database (DB) using data that is generated as the treated cases of OSAS using feature information are accumulated. The image and diagnostic information output unit 140 may extract the treated cases, using the variables included in the feature information extracted by the feature extraction unit 130, from the learning-based database, and may provide diagnostic information required for treatment (for example, information about the establishment of a plan for surgery based on the location of obstruction of the upper airway and the condition of the obstruction or information about prediction about the level of improvement of OSAS after use of an MAD) to a user. Furthermore, the image and diagnostic information output unit 140 may provide the conductivity images restored by the feature extraction unit 130, in addition to the feature information.

By using the device 100 for imaging and diagnosing the condition of obstruction of the upper airway by using EIT, configured as described above, the presence, level, frequency, shape, area, etc. of obstruction of the upper airway can be effectively extracted over the course of several hours in a natural sleep state, and also the inconvenience of an examinee can be minimized.

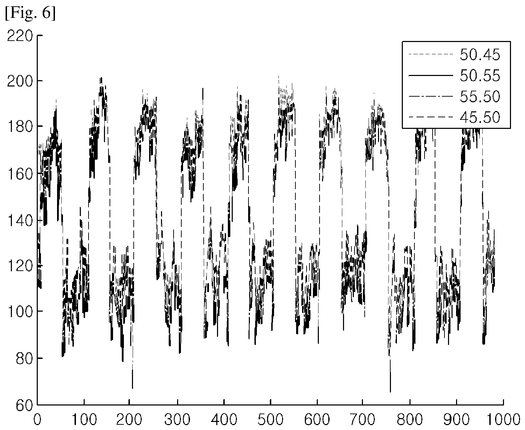

Fig. 5 shows conductivity images restored from data measured at the times at which the upper airway was opened and obstructed, respectively, and Fig. 6 is a graph showing the time-varying data of conductivity values restored from voltage data measured in a situation in which opening and obstruction of the upper airway were repeated.

While the present invention has been described with reference to the illustrated embodiments above, these embodiments are merely examples. It will be apparent to those having ordinary knowledge in the art to which the present invention pertains that various modifications, variations and other equivalent embodiments can be made within the gist and scope of the present invention. Accordingly, the true technical protection range of the present invention should be defined based on the technical spirit of the present invention disclosed in the attached claims.

### Industrial Applicability

The present invention relates to a device for imaging and diagnosing the condition of obstruction of the upper airway, which can image the condition of a change in the upper airway of an OSAS patient occurring during natural sleep by using EIT and can extract information required for diagnosis. The device for imaging and diagnosing the condition of obstruction of the upper airway by using EIT according to the present invention does not require high costs, enables repeated examination to be conducted without the risk of exposure to radioactive rays, and enables examination to be performed over a prolonged period of time in an actual natural sleep state, thereby accurately diagnosing the condition of obstruction of the upper airway.

## Claims

1. A device for imaging and diagnosing a condition of obstruction of an upper airway by using electrical impedance tomography, the device comprising:
a conductivity signal measurement unit configured to measure a distribution of electric potentials that changes in accordance with a change in a shape of the upper airway;
a feature extraction unit configured to restore conductivity images of an upper airway by using the measured distribution of electric potentials and extract feature information from the restored conductivity images; and
an image and diagnostic information output unit configured to display the extracted feature information or restored conductivity images.

2. The device of claim 1, further comprising a multichannel electrode interface including multichannel electrodes attached onto face surfaces around a region of the upper airway;
wherein the conductivity signal measurement unit measures the distribution of electric potentials by applying current via the multichannel electrode interface and measuring voltage induced by the applied current.

3. The device of claim 1, wherein the feature extraction unit extracts a change in an area of a region of the upper airway or a geometrical feature of the region of the upper airway by using a feature extraction algorithm.

4. The device of claim 1, wherein the feature extraction unit performs removal of noise by using the data on the change in the distribution of electric potentials measured through monitoring.

5. The device of claim 4, wherein the feature extraction unit performs the removal of noise by using a pattern analysis of interval data or an average value of the feature information.

6. The device of claim 1, wherein the conductivity signal measurement unit measures the distribution of electric potentials at high speed so that the conductivity images of the upper airway are restored at a rate of 50 to 100 images per second.

7. The device of claim 1, wherein the image and diagnostic information output unit synchronizes the feature information or the conductivity images, corresponding to a plurality of layers, with each other, and then provides them.

8. The device of claim 1, wherein the image and diagnostic information output unit provides diagnostic information corresponding to the feature information based on a learning-based database that stores data on treatment cases of obstructive sleep apnea syndrome.

9. A device for imaging and diagnosing a condition of obstruction of an upper airway by using electrical impedance tomography, the device comprising:
a multichannel electrode interface; and
an imaging device configured to restore conductivity images by using current-voltage data transferred from the multichannel electrode interface;
wherein the multichannel electrode interface comprises:
multichannel electrodes attached onto face surfaces around a region of the upper airway; and
a protective gear configured in a shape that surrounds outsides of the multichannel electrodes.

10. The device of claim 9, wherein the multichannel electrodes include conductive fiber electrodes manufactured based on Ag-plated elastic fiber or PVDF nanofiber web.

11. The device of claim 9, wherein the multichannel electrodes are arranged in a three-dimensional array including a plurality of layers.
